(19) Europäisches Patentamt

European Patent Office

Office européen des brevets

(11) **EP 0 602 039 B1**

(12) **EUROPEAN PATENT SPECIFICATION**

(45) Date of publication and mention
of the grant of the patent:
**18.09.1996 Bulletin 1996/38**

(21) Application number: **92908739.3**

(22) Date of filing: **13.04.1992**

(51) Int Cl.$^6$: **G01L 1/12**, G01B 7/24,
G01N 27/00, G01N 27/72

(86) International application number:
**PCT/NO92/00068**

(87) International publication number:
**WO 92/18839 (29.10.1992 Gazette 1992/27)**

(54) **METHOD FOR MEASURING MECHANICAL STRESSES AND FATIGUE CONDITIONS IN STEEL**

VERFAHREN ZUR MESSUNG DER MECHANISCHEN SPANNUNG UND DER
ERMÜDUNGSBEDINGUNGEN VON STAHL

PROCEDE DE MESURE DES CONTRAINTES MECANIQUES ET DES CONDITIONS DE FATIGUE
DANS L'ACIER

(84) Designated Contracting States:
**AT BE CH DE DK ES FR GB IT LI NL SE**

(30) Priority: **12.04.1991 NO 911437**

(43) Date of publication of application:
**22.06.1994 Bulletin 1994/25**

(73) Proprietor: **HOGNESTAD, Harek**
**N-1344 Haslum (NO)**

(72) Inventor: **HOGNESTAD, Harek**
**N-1344 Haslum (NO)**

(74) Representative: **Rackham, Stephen Neil**
**GILL JENNINGS & EVERY,**
**Broadgate House,**
**7 Eldon Street**
**London EC2M 7LH (GB)**

(56) References cited:
**NO-B- 150 136**          **US-A- 4 528 856**
**US-A- 5 012 189**

- **NDT International, Vol. 19, No. 2, April 1986,**
  **BOSE M.S.C.: "A study of fatigue in**
  **ferromagnetic materials using a magnetic**
  **hysteresis technique"**
- **Controller HMSO, 1987 (London) LUGG M.C.:**
  **"The effect of stress on the ACFM technique",**
  **see the whole document.**
- **EP-B1-105893**

## Description

The present invention relates to a method for obtaining information regarding the condition of steel structures by means of a measuring method which can be adopted for detecting cracks, material loss (for example corrosion), mechanical stresses and fatigue.

Large steel structures are often subjected to varying loads. For safety reasons it is important to inspect and monitor such structures in order to discover conditions which can develop into serious defects. Comprised herein are, inter alia, mechanical stresses which can arise unintentionally both during construction, (for example in or adjacent weld seams) and during operation because of uncontrollable conditions.

For measuring relative mechanical stresses strain gauges or other means are often employed for detecting small dimensional changes in the steel (elongation measurements). By employment of such methods changes are measured from the instant when the arrangement was installed.

When absolute mechanical stresses are concerned, the possibilities are more limited. It is known, however, that measurement and analysis of Barkhausen noise can render information about mechanical stresses (reference 1). Moreover it is to be noted that mechanical stresses lead to changes in magnetic properties and use of this phenomenon has been attempted as a basis for measuring mechanical stresses in steel (reference 2).

The present invention is based on the measurement of magnetic properties in steel by means of a particular measurement method which takes Norwegian patent No. 150,136 (reference 3) as a starting point. This patent is directed to the detection of cracks and material loss in metal structures. The further development to be described below is primarily directed to the measurement of mechanical stresses and fatigue conditions in steel, but can also be of significance in connection with improving the capability of detecting cracks and material loss.

The above Norwegian patent mainly describes a method which is based on the measurement of voltage drop (potential drop) when an electric current is applied. The voltage drop is measured between a number of contact points both when the structure is in a preferably known initial condition and during operation. The voltage drops measured are compared with each other and form the basis for detecting changes in the condition of the structure, in the first place cracks and material loss.

The current applied is pulse shaped. Upon each current step the voltage drop measured will follow a transient curve because of the skin effect, which in the above patent is referred to as disturbances. Therefore, the application of current takes place at a low pulse frequency so that the transient portion of the voltage drop has decayed before measurement takes place. Thus, according to the patent a prejudice exists against the idea of considering the transient portion of the voltage drop curve as informative as far as possible defects or changes in the structure are concerned.

On the contrary, the present invention is based on the idea of deriving information from the transient curve portion of the voltage or potential drop. During the time when the potential drop over a set of contact points varies after each step of the applied current (excitation current) the potential drop is therefore measured by repeated sampling so that there is obtained a picture of the whole transient curve in the form of a series of number values which can be further processed in a computer.

In order to obtain good accuracy the repeated sampling can be performed over several periods of the excitation current, so that on basis of a larger amount of data accurate average values can be calculated. More or less advanced algorithms can be employed for computing accurate average values.

The transient potential drop as a function of time contains information with respect to the electrical and magnetic properties of the steel. When the transient curve after a time interval has completely decayed (the potential drop is then stationary) the level of the potential drop is given by the electric resistivity of the steel, which in turn is influenced by cracks and material loss

As already indicated the first portion of the transient curve is mainly related to the magnetic properties of the steel which in turn is influenced by mechanical stresses.

In the points listed below it is indicated how magnetic properties of steel are influenced by mechanical stress. The basic physical phenomena and more thorough discussions thereof are available in the literature (reference 5).

.   The shape or character of both the initial, the anhysteretic and the hysteresis magnetisation curve is changed upon the application of mechanical stress.

.   At a given constant magnetic field strength an applied mechanical stress will bring the initial and the anhysteretic magnetisation curves to approach each other.

.   The change of magnetisation as a consequence of the changed mechanical stress, is not restored when the mechanical stress is reduced to the initial level. Only upon demagnetisation by applying a magnetic field with a gradually diminishing amplitude to zero, the magnetisation curves will return to their original shape.

It has also been shown that the magnetic properties of steel can be influenced during a fatigue process (reference 4).

.   The character of the magnetisation curves is changed during a fatigue process. These changes cannot be restored by demagnetisation.

The measurement of relative mechanical stresses in steel can be based on the measurement of the magnetisation (and thus the character of the magnetisation curves) under given conditions, for example with reference to the anhysteretic magnetisation curve at a given field strength, and comparison with earlier measurments. The method described in Norwegian patent No. 150.136 can then be employed. Measurements are then performed also at a location which is not subjected to stress or strain (denoted reference measurement in the above patent). In addition thereto possibilities of demagnetisation are incorporated.

A similar method can be employed for measuring the maximum stress to which the steel has been subjected since the last measurement. Then the property mentioned, i.e. that a magnetisation change is not restored or reset until upon demagnetisation, is utilized.

Measurement of absolute mechanical stresses in steel can be based on the fact that the initial magnetisation curve and the anhysteretic magnetisation curve will be changed upon the application of mechanical stress. The transient voltage drops, which are influenced by the character of the magnetisation curves, are measured with reference to both the initial and the anhysteretic magnetisation curve, and is compared with each other. In the alternative the two measurements can be made with reference to the hysteresis and the anhysteretic magnetisation curve.

When there is no mechanical stress in the steel the fatigue condition can be measured in a similar way as absolute mechanical stresses. It has not been fully clarified, however, how the various magnetisation curves (and thereby the transient potential drop curves) are changed in relation to each other during a fatigue process. It is to be expected, however, that fatigue will lead to certain characteristic features in the transient potential drop curves, which presumably can be recognized when the curves are subjected to a closer analysis.

Thus the method for measuring mechanical stresses and fatigue conditions in steel, whereby a changing electric current is applied to the steel through a first pair of contact points, according to the invention is characterized by measuring the transient potential drop which is caused thereby between a second pair of contact points, by repeated sampling, and that the results of several measurements are further processed in order to determine the mechanical stress or the fatigue condition in the steel.

The method can be employed directly on steel structures. Current injection and measurement of transient potential drop is then performed directly on the steel structure. The method can also be employed in sensors. Current injection and measurement of transient potential drop is then performed for example in a beam which is attached to the structure so that it is subjected to the mechanical strain concerned.

A main concept utilized by the present invention as already explained, is the phenomenon that the electrical potential drop curves (transient curves) which arise between a pair of contact points as a consequence of current pulses applied, change when the mechanical stress is changed. The potential drop curves are related to the skin effect, which in turn is related to the permeability of the steel. (As known, relative permeability is a parameter which indicates the capability of magnetisation when a magnetic field is applied).

When applying a current step, for example in a steel plate, the current density at the surface will be high immediately after the current step, and decreases gradually to a stationary value. The potential drop measured between two points thus will reflect the current density at the surface, which is a function of time having high values initially and decreasing to a stationary value corresponding to the electrical resistivity between the measurement point. Thus, the transient potential drop renders information about both the permeability and the conductivity of the material, which according to the discussions above can be influenced by mechanical stress/ fatigue condition as well as material loss/corrosion. For the time being it has not been verified to which degree the conductivity varies during a fatigue process before cracks can be detected by means of common NDT methods. Possibly such changes may be due to a large number of microcracks.

Further features of the invention as far as the measurement of absolute mechanical stresses is concerned, are stated in the claims, and shall be explained in the following description with reference to the figures of drawings.

Fig. 1    shows an arrangement for measuring absolute stresses at a section of a steel plate.

Fig. 2    shows a current step and potential drop A(t) as a function of time.

Fig. 3    shows two potential drop curves and a deviation curve being calculated.

Fig. 4    shows deviation curves measured for a steel beam which was subjected to various degrees of mechanical stress.

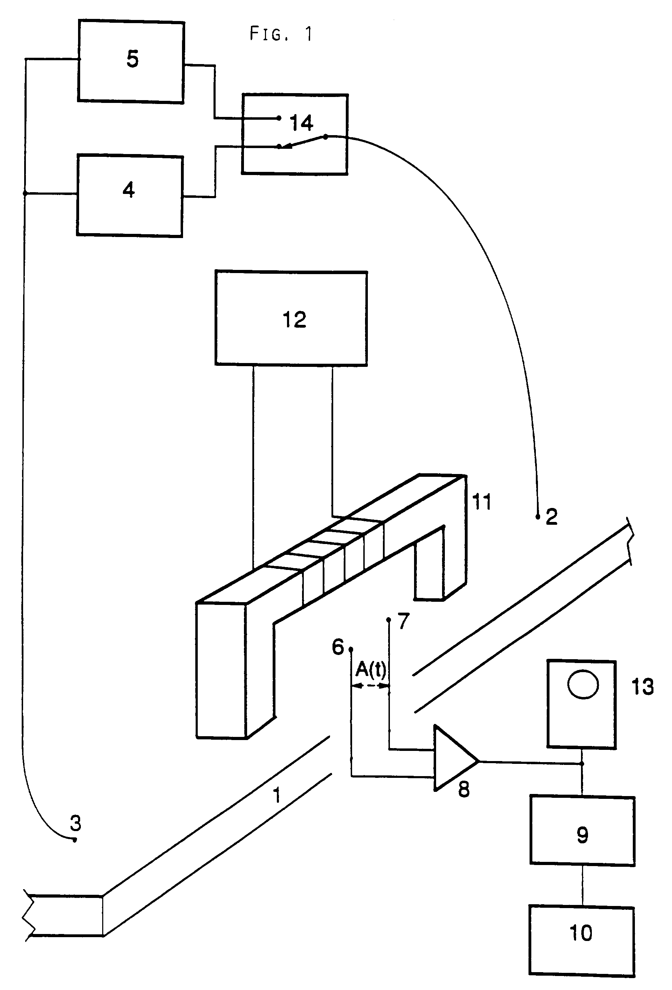

To the steel plate 1 in Fig. 1 current pulses (excitation current) is applied by means of the source of current 4 through contact points 2, 3. When required a source of current 5 alternatively can be connected for demagnetisation, by means of a switch 14. By means of this source of current there is applied an alternating current with a diminishing amplitude down to zero. The potential drop A(t) which is measured between measurement points 6,7 is led to the amplifier 8. The output signal from the amplifier is digitized in an A/D converter 9 for further processing in a computer 10. The analogue signal from the amplifier can be led to an oscilloscope 13 for visual observation, but more often, however, the changes brought about will be so small that it is difficult to observe them on the oscilloscope screen. By means of an electromagnet 11 the steel can be magnetized at the location where the potential drop is measured. The strength of the magnetic field applied can be selected as a desired magnitude by means of an adjustable source of current 12.

The current sources 4, 5 and 12 as well as the switch 14 can be controlled from the computer. This control function has not been shown in Fig. 1.

Demagnetization by means of the current source 5 alternatively can be performed by delivering the current to an electromagnet instead of switching to the injection points 2, 3 for the excitation current. The switch 14 is then superfluous. As a third alternative the current from the demagnetization source 5 can be injected into the steel through contact points being only employed for this purpose. Also in this case the switch 14 is superfluous.

Injection points 2, 3, measurement points 6,7 and the electromagnet 11 can be built together as a portable unit.

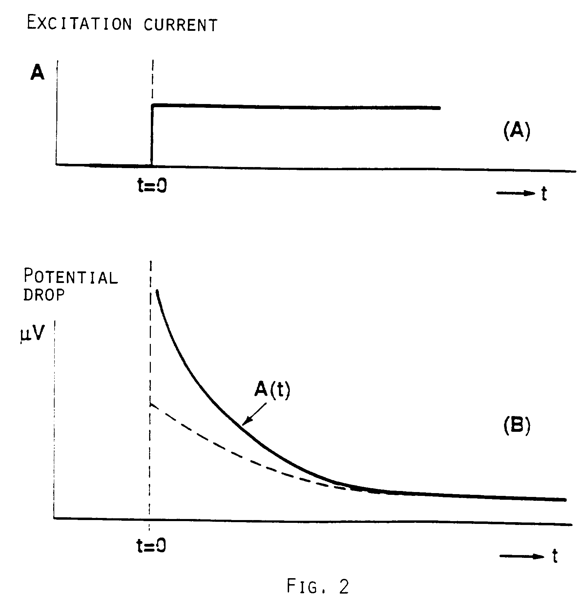

In Fig. 2 a typical current step (A) and potential drop (B) are shown with ideal curve shapes. In actual practice the current step will have a certain rise time, and the signal (potential drop) will have an additional component due to electric induction between measuring leads and the conductor loop which form the injection circuit. It is also possible to let the current step start from a negative value.

The potential drop curve according to its nature is a Bessel function. It starts from theoretical infinity, and has a dominating time constant which means that the curve will by and by approach an exponential function (dotted curve). The stationary value which A(t) is approaching, is exclusively determined by the ohmic resistance between the measurement points. The time constant of the exponential function mentioned, is determined by geometrical relationships as well as conductivity and permeability.

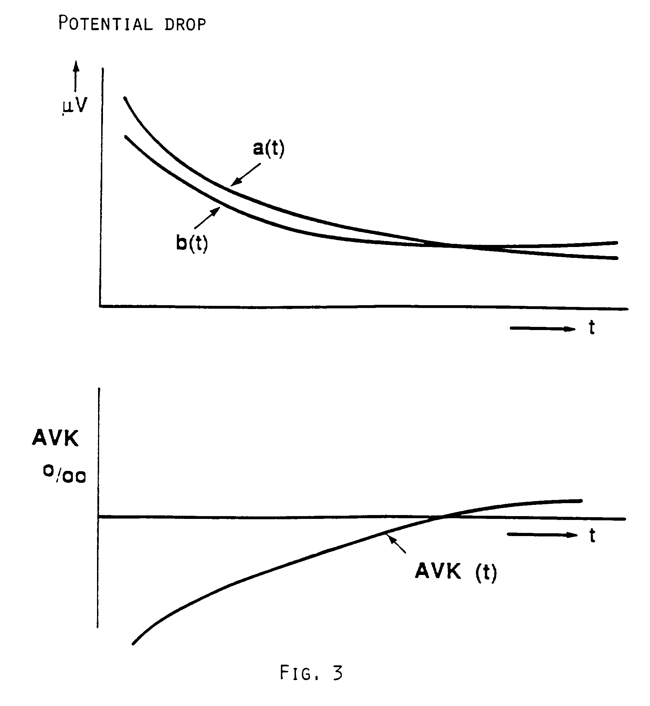

If the potential drop measured at the opposite side of a steel plate in relation to that side where the current step is applied, A(t) will start from zero, and will increase until the stationary value is reached. The measurements will always be based on the comparison of at least two curves as a function of time. Fig. 3 illustrates how, on the basis of two curves a(t) and b(t), a deviation curve, AVK(t), is computed from the difference between the two curves. The deviation curve is here computed as

$$AVK(t)=(\frac{b(t)}{a(t)} -1)1000$$

but also other algorithms can be utilized in order to characterize the deviation between various curves plotted as a function of time.

As an additional explanation referring to Fig. 1 a sequence is described below explaining how the arrangement shown can be employed for measuring transients with reference to the intial and the anhysteretic magnetization curve:

1) The current from the current source 12 to the electromagnet 11 is set to zero.

2) The current injection points 2, 3 are connected to the current source 5 by means of switch 14. The current source 5 is activated for demagnetization.

3) By means of switch 14 the current injection points 2, 3 are connected to current source 4 which applies a pulse shaped current to the steel.

4) The current from current source 12 to electromagnet 11 is continually increased to the desired magnitude. The steel is now magnetized with reference to the initial magnetization curve.

5) The transient potential drop between measurement points 6, 7, i.e. a(t), is measured and stored in the computer 10.

6) The above point 2 is repeated. After this the steel is magnetized with reference to the anhysteretic magnetization curve.

7) The above point 3 is repeated.

8) The transient potential drop between measurement points 6, 7, i.e. b(t), is measured and stored in computer 10.

9) Computer 10 computes AVK(t) according to the expression given above.

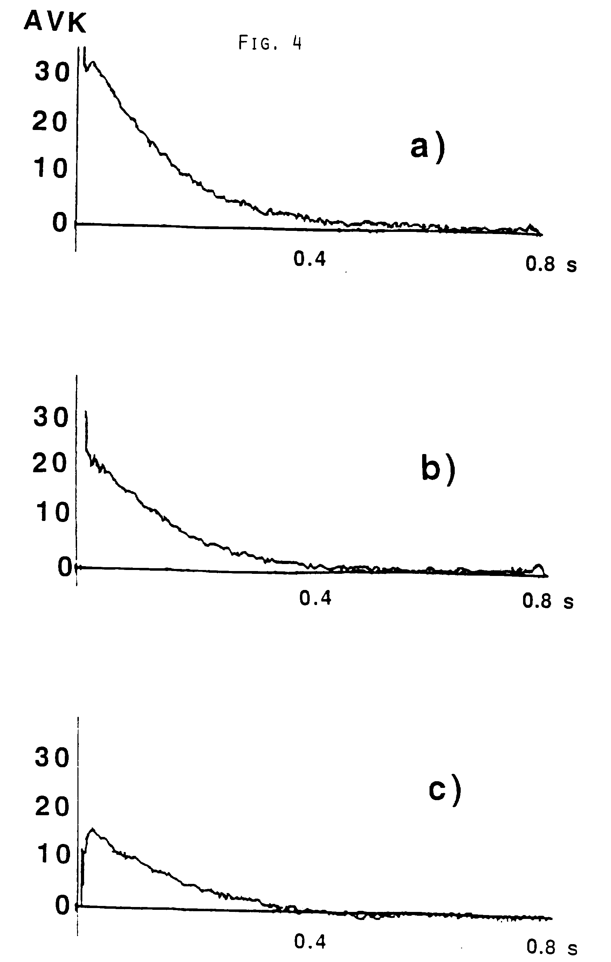

Examples of deviation curves computed and printed from a computer, are shown in Figs. 4a, 4b and 4c where the applied mechanical stress on a steel beam is 0, 100 and 300 MPa tensional load respectively. The magnetic field applied in this case is 1,4 kA/m. The horizontal scale in Fig. 4 is 0 to 0.7 sec.

Similar measurements can be made at a different field strength and with reference to the hysteresis and the anhysteretic magnetization curve as the case may be. If desired it is also possible to compute deviation curves with reference to the initial and the hysteresis magnetization curves.

From experience as to the character of the deviation curves at different mechanical stresses it will be possible to determine the unknown stress condition.

In a similar way deviation curves can render information with respect to the fatigue condition in the steel, and thus form a basis for computing remaining lifetime before failure in a fatigue process.

LITERATURE REFERENCES

(1) Rautioaho, R.H., and Karjalinen, L.P.: "Application of Barkhausen noise measurements to residual stress analysis in structural steels". Proc. Scandinavian Symposium in Materials Science, 20-21 June 1983, Oulu Finland, pp 179-185.

(2) Lugg M. C.: "The effect of stress on the ACFM technique". Controller (C) HMSO, London 1987, 10 pages.

(3) Hognestad H.: "Method and arrangement for monitoring large structures of metal" Norwegian Patent No. 150.136 (EP-B)-105893, 13 pages).

(4) Bose M. S. C. : "A study of fatigue in ferromagnetic materials using a magnetic hysteresis technique" NDT International Vol. 19 No. 2, April 1986, pp 83-87.

(5) Jiles D.C. and Aderton D.L.: "Theory of the magnetisation process in ferromagnets and its application to the magnetomechanical effect". J. Phys. Appl., 17 (1984) pp 1265-1281.

## Claims

1. Method for measuring mechanical stresses and fatigue conditions in steel (1), including the step of changing the electric current applied to the steel through a first pair of contact points (2, 3), characterized in that the transient potential drop thereby brought about between another pair of contact points (6, 7) is measured by repeated sampling and that the results of several measurements are further processed (8 - 10) for determining the mechanical stress or the fatigue condition in the steel.

2. Method according to claim 1, whereby the steel is magnetized and demagnetized by means of arrangements known per se, characterized in that these arrangements are employed for magnetizing the steel at selected points of the initial, the anhysteretic and the hysteresis magnetization curve at the location where the measurements take place, and that the transient potential drop is measured with reference to one or more of said magnetization curves and that the result of these measurements is utilized for determining the mechanical stress and/or the fatigue condition in the steel.

3. Method according to claim 1 or 2, further characterized in that a repeated sampling of the transient potential drop is carried out over several periods of the change in the applied current and that on the basis of these measurements average values relating to the transient potential drop are computed.

4. Method according to one of claims 1 to 3, further characterized in that the steel when required is demagnetized by the application of an alternating current having a gradually diminishing amplitude down to zero, through said first pair of contact points which is employed for the change in the applied current.

5. Method according to one of claims 1 - 4, characterized in that both initial and later measurements are carried out at a location which is subjected to mechanical load and a location which is not loaded and that the results of these measurements are utilized for determining the change in the mechanical stress and/or the fatigue condition in the steel in relation to what was the case at the time of the initial measurement.

6. Method according to one of claims 1 - 4, characterized in that both initial and later measurements are carried out at a location which is subjected to mechanical load and a location which is not loaded and that the results of these measurements are utilized for determining the maximum mechanical stress through which the steel has been subjected since the time of the initial measurement.

## Patentansprüche

1. Verfahren zum Messen mechanischer Beanspruchungen und Ermüdungserscheinungen von Stahl (1), das den Schritt des Änderns des auf den Stahl mittels eines ersten Paars von Kontaktpunkten (2,3) beaufschlagten elektrischen Stroms beinhaltet, dadurch gekennzeichnet, daß der dadurch bewirkte transiente Potentialabfall zwischen einem anderen Paar von Kontaktpunkten (6,7) durch wiederholtes Abtasten gemessen wird und daß die Ergebnisse von mehreren Messungen weiterverarbeitet werden (8 bis 10) zum Bestimmen der mechanischen Beanspruchung oder der Ermüdungserscheinung des Stahls.

2. Verfahren nach Anspruch 1, bei dem der Stahl mittels an und für sich bekannter Anordnungen magnetisiert und demagnetisiert wird, dadurch gekennzeichnet, daß diese Anordnungen zum Magnetisieren des Stahls an ausgewählten Punkten der ursprünglichen, der anhysteretischen und der Hysteresis-Magnetisierungskurve an derjenigen Stelle, an der die Messungen stattfinden, verwendet werden, und daß der transiente Potentialabfall unter Bezug auf eine oder mehrere der genannten Magnetisierungskurven gemessen wird und daß das Ergebnis dieser Messungen zum Bestimmen der mechanischen Beanspruchung und/oder der Ermüdungserscheinung des Stahls verwendet wird.

3. Verfahren nach Anspruch 1 oder 2, weiter gekennzeichnet dadurch, daß ein wiederholtes Abtasten des transienten Potentialabfalls über mehrere Perioden der Änderung des beaufschlagten Stroms durchgeführt wird und auf der Basis dieser Messungen Mittelwerte bezüglich des transienten Potentialabfalls berechnet werden.

4. Verfahren nach einem der Ansprüche 1 bis 3, dadurch gekennzeichnet, daß der Stahl, wenn erforderlich, durch Beaufschlagung eines eine sich allmählich bis auf Null verringernde Amplitude aufweisenden Wechselstroms demagnetisiert wird, der über das erste Paar von Kontaktpunkten beaufschlagt wird, die zur Änderung des beaufschlagten Stroms verwendet werden.

5. Verfahren nach einem der Ansprüche 1 bis 4, dadurch gekennzeichnet, daß sowohl anfängliche als auch spätere Messungen an einer mechanischer Beanspruchung ausgesetzten Stelle und einer nicht beanspruchten Stelle ausgeführt werden und daß die Ergebnisse dieser Messungen verwendet werden zur Bestimmung der Änderung der mechanischen Beanspruchung und/oder der Ermüdungserscheinung des Stahls bezüglich des Zustands im Zeitpunkt der anfänglichen Messung.

6. Verfahren nach einem der Ansprüche 1 bis 4, dadurch gekennzeichnet, daß sowohl anfängliche als auch spätere Messungen an einer einer mechanischen Beanspruchung ausgesetzten Stelle und einer nicht beanspruchten Stelle ausgeführt werden und daß die Ergebnisse dieser Messungen zur Bestimmung der maximalen mechanischen Beanspruchung verwendet werden, der der Stahl seit dem Zeitpunkt der anfänglichen Messung unterworfen worden ist.

**Revendications**

1. Procédé de mesure de contraintes mécaniques et de conditions de fatigue dans de l'acier (1), comprenant une étape de changement du courant électrique appliqué à l'acier par une première paire de points de contact (2, 3), caractérisé en ce que la chute transitoire de potentiel ainsi provoquée entre une autre paire de points de contact (6, 7) est mesurée par échantillonnage répété, et en ce que les résultats de plusieurs mesures subissent un traitement supplémentaire (8-10) pour la détermination de la contrainte mécanique ou de la condition de fatigue dans l'acier.

2. Procédé selon la revendication 1, dans lequel l'acier est aimanté et désaimanté par des ensembles de type connu, caractérisé en ce que ces ensembles sont utilisés pour l'aimantation de l'acier en des points choisis de la courbe initiale d'aimantation avec hystérésis et sans hystérésis à l'emplacement auquel les mesures sont réalisées, en ce que la chute de potentiel transitoire est mesurée par rapport à une ou plusieurs des courbes d'aimantation, et en ce que le résultat de ces mesures est utilisé pour la détermination de la contrainte mécanique et/ou de la condition de fatigue dans l'acier.

3. Procédé selon la revendication 1 ou 2, caractérisé en outre en ce qu'un échantillonnage répété de la chute de potentiel transitoire est réalisé sur plusieurs périodes du changement du courant appliqué, et en ce que, d'après ces mesures, des valeurs moyennes relatives à la chute de potentiel transitoire sont calculées.

4. Procédé selon l'une des revendications 1 à 3, caractérisé en outre en ce que l'acier est le cas échéant désaimanté par application d'un courant alternatif ayant une amplitude qui diminue progressivement jusqu'à une valeur nulle, à l'aide de la première paire de points de contact utilisée pour le changement du courant appliqué.

5. Procédé selon l'une des revendications 1 à 4, caractérisé en ce que les mesures initiales et ultérieures sont exécutées à un emplacement qui est soumis à une charge mécanique et à un emplacement qui n'est pas sous charge, et en ce que les résultats de ces mesures sont utilisées pour la détermination du changement de la contrainte mécanique et/ou de la condition de fatigue dans l'acier par rapport aux résultats obtenus au moment de la mesure initiale.

6. Procédé selon l'une des revendications 1 à 4, caractérisé en ce que les mesures initiales et ultérieures sont réalisées à un emplacement qui est soumis à une charge mécanique et à un emplacement qui n'est pas sous charge, et en ce que les résultats de ces mesures sont utilisés pour la détermination de la contrainte mécanique maximale à laquelle l'acier a été soumis depuis le moment de la mesure initiale.

FIG. 1

EXCITATION CURRENT

**A**

(A)

t=0 ⟶ t

POTENTIAL
DROP

μV

A(t)

(B)

t=0 ⟶ t

FIG. 2

POTENTIAL DROP

$\mu V$

a(t)

b(t)

t

AVK
o/oo

AVK (t)

t

FIG. 3

FIG. 4

a)

b)

c)